**Europäisches Patentamt**

**European Patent Office**

**Office européen des brevets**

(19)

(11) Publication number : **0 428 017 B1**

## EUROPEAN PATENT SPECIFICATION

(12)

(45) Date of publication of patent specification :
**23.02.94 Bulletin 94/08**

(21) Application number : **90120943.7**

(22) Date of filing : **31.10.90**

(51) Int. Cl.⁵ : **C09J 193/00,** A61L 15/58,
// (C09J193/00, 153:00)

(54) **Hot melt adhesive composition.**

(30) Priority : **16.11.89 JP 298708/89**

(43) Date of publication of application :
**22.05.91 Bulletin 91/21**

(45) Publication of the grant of the patent :
**23.02.94 Bulletin 94/08**

(84) Designated Contracting States :
**BE DE ES FR GB IT SE**

(56) References cited :
**EP-A- 0 104 005**
**EP-A- 0 302 394**
**DE-A- 3 306 438**

(73) Proprietor : **National Starch and Chemical**
**Investment Holding Corporation**
**501 Silverside Road**
**Wilmington, Delaware 19809 (US)**

(72) Inventor : **Sugie, Masaharu**
**605-206, Tomobuchicho 1-chome**
**Miyakojima-ku, Osaka 535 (JP)**
Inventor : **Okkotsu, Masao**
**9-8, Hanazonocho**
**Nishinomiya-shi, Hyogo 633 (JP)**

(74) Representative : **Hagemann, Heinrich, Dr. et al**
**Patentanwälte Hagemann & Kehl Postfach**
**860329**
**D-81630 München (DE)**

## Description

This invention concerns a hot melt adhesive composition for preventing slipping which is applied to parts of the surfaces of paper diapers of the type which require diaper covers, sanitary napkins, etc., in order to prevent the said diapers or napkins from slipping inside diaper covers or underwear.

Hot melt adhesives with synthetic rubbers as base polymers, to which tackifying resins and plasticizing oils are added, have been used up to now as slipping-prevention adhesives, which are applied to parts of the surfaces of paper diapers and napkins. Among the important performances required of such hot melt adhesives are: (1) that they be soft, white or colorless, and odorless; (2) that they have excellent initial adhesiveness to the diaper cover or underwear to which they are to be adhered, and that this adhesiveness be excellent from low to high temperatures; and (3) that no adhesive remains on the diaper cover or underwear when the paper diaper or napkin is removed after use. However, among conventional hot melt adhesives, none satisfy all of these requirements in a superior way. For example, the hot melt adhesives which have been principally used up to now have had the difficulty that they are compounded with large quantities of plasticizing oil, since their hand [i.e., feel] has been considered important, but when used with napkins, etc., low-molecular-weight substances in the adhesive exude in liquid form when the adhesive atmosphere [atmosphere around the adhesive] is warmed to a high temperature by the body heat, so that in the end the underlayer (underwear, etc.) is dirtied. In contrast to this, the use of styrene-ethylene-butylene-styrene block copolymers (SEBS) has recently come to be preferred for the synthetic rubber base. These SEBS can accept an extremely large quantity of plasticizing oil in their polymer skeletons, compared to styrene-isoprene-styrene block copolymers (SIS) and styrene-butadiene-styrene copolymers (SBS), so that they have the advantage that a very small quantity of oil exudes from the base polymer even when the temperature rises. However, when they are used for long periods of time in body temperature atmospheres, they have the drawback that the hot melt adhesive gradually softens and penetrates into the underlayer to which [the napkin, etc.] is adhering; the apparent adhesive force greatly increases, and at the same time the cohesive force (the force which resists peeling off) is reduced, so that the adhesive layer suffers cohesion breakdown when the napkin, etc. is removed, and some adhesive remains on the underlayer. In order to solve this problem, the best approach is to reduce the quantity of the plasticiziing oil in the composition, but if the quantity of plasticizing oil is small, the initial adhesive force is greatly reduced, and at the same time the problem arises of the adhesive becoming hard and causing slipping to occur easily during use. Therefore, the quantity of plasticizing oil in the composition is adjusted to minimize both of these drawbacks, but at present a satisfactory hot melt adhesive for preventing slipping has not necessarily been obtained.

The nearest or the relevant state of the art is disclosed in EP-A-104 005 and DE-A-3 306 438. EP 0 104 005 discloses a hot melt pressure-sensitive adhesive, suitable for sanitary products, which is selected to exhibit critical rheological properties which manifest themselves into the desired perfomance criteria of tenacious bonding and clean release. This known composition can comprise an A-B-A block copolymer, preferably comprising a mid-block of polyolefins and an endblock of polystyrene. The mid-block preferably comprises copolymers of ethylene and butylene. DE-A-33 06 438 relates to an adhesive for bonding articles on the basis of EPDM with other articles which adhesive comprises a solution of a) a SEBS-block copolymer-rubber, b) 50-300 parts by weight phr aliphatic hydrocarbon resin, c) 10-150 parts by weight phr aromatic hydrocarbon resin and zinc oxide, magnesium oxide and antioxidants.

The purpose of the present invention is to provide a hot melt adhesive composition which combines a satisfactory slippage preventing effect with a satisfactory separating ability from the underlayer, which could not coexist previously, and also has a soft hand.

In order to accomplish the above purpose, the hot melt adhesive composition of this invention contains 5-30 wt.% styrene-ethylene-propylene-styrene block copolymer (SEPS), the styrene content of the SEPS copolymer being from 10-40 wt.%, 5-30 wt.% styrene-ethylene-butylene-styrene (SEBS) block copolymer, the styrene content of the SEBS-copolymer being from 10-35 wt.%, 40-70 wt.% hydrogenated tackifying resin ingredient, and 10-50 wt.% plasticizing oil ingredient.

That is, the inventors performed a series of studies in order to obtain adhesive compositions which would combine a satisfactory slippage-preventing effect with a satisfactory separating ability from the underlayer, which could not coexist previously. As a result, they discovered that, if specific quantities of SEPS and SEBS were compounded as the synthetic rubber ingredient which serves as the base polymer of the hot melt adhesive composition, a specific quantity of a hydrogenated resin was compounded as a tackifying resin ingredient, and a specific quantity of a plasticizing oil was also compounded, it was possible to obtain a hot melt adhesive with excellent slippage-preventing effect and underlayer-separating ability at the same time; thus, this invention was achieved.

The hot melt adhesive composition of this invention contains SEPS and SEBS synthetic rubber ingredients, a tackifying resin ingredient, and a plasticizing oil ingredient, each in a specific proportion.

The aforementioned SEPS is a copolymer which contains a mixture of an ethylene structure and a propylene structure between styrene polymer blocks on the ends; in this two-phase structure, the styrene phase functions as hard segments and the ethylene-propylene phase as a soft segment. The SEPS ordinarily has the same oil-retaining ability as SEBS, or a greater ability, and the plasticizing oil does not exude over time. Moreover, it also has excellent initial adhesive force, without a reduction of the cohesive force, and a stable adhesive force in the range from room temperature to the body temperature region can be obtained. At the same time, one can also obtain a soft hand. This SEPS can be obtained simply by, for example, hydrogenating the isoprene polymer ingredient of a styrene-isoprene-styrene block copolymer (SIS). However, when used in this invention, the aforementioned SEPS has a styrene content of 10-40 wt % If the styrene content is less than 10 wt %, the cohesive force tends to be markedly reduced, whereas if it is over 40 wt % a tendency is seen for the stability of the adhesive force from room temperature to the body temperature range to become poorer. The quantity of the aforementioned SEPS in the composition must be 5-30 wt % of the whole composition; a preferable proportion is 5-20 wt %, and it is still more preferable for it to be 5-15 wt %. If the proportion of the SEPS is too small, the adhesiveness is inferior, and if it is too great, the cohesive force is reduced.

Moreover, the SEBS used together with the aforementioned SEPS is a copolymer which is formed by mixing an ethylene structure and a butylene structure between styrene polymer blocks on the ends; it has a two-phase structure in which the styrene phase functions as hard segments and the ethylene-butylene phase functions as a soft segment. This SEBS can be obtained simply by, for example, hydrogenating the butadiene polymer part of a styrene-butadiene-styrene block copolymer (SBS). However, when used in this invention, the aforementioned SEBS has a styrene content of 10-35 wt %. If the styrene content is less than 10 wt %, a tendency is seen for the cohesive force in the body temperature region to become poorer, whereas if it is greater than 35 wt %, a tendency is seen for the adhesive force at room temperature to become extremely poor. The quantity of the aforementioned SEBS must be 5-30 wt % of the whole composition; a preferable proportion is 7-25 wt %, and it is still more preferable for it to be 10-20 wt %. If the proportion is too great, the adhesive force is reduced and the hand becomes hard; in addition, the melt viscosity is raised, and the application operability becomes poor.

On the other hand, the tackifying resin ingredient used in this invention has a low molecular weight; it increases the wettability of the composition and at the same time imparts an initial adhesive force; it must reduce the viscosity of the composition as a whole and improve the application operability of the adhesive. Moreover, it is desirable for it to be colorless or white and odorless. Therefore, in this invention, it is especially necessary to use a hydrogenated resin in this invention. That is, since hydrogenated resins have excellent miscibility with the aforementioned SEPS and SEBS, a wide range of adhesiveness is obtained, from low temperatures to high temperatures, and at the same time its thermal stability is excellent, since it has no double bonds in its chemical structure. As such tackifying resin ingredients, hydrogenated aromatic petroleum resins, hydrogenated aliphatic petroleum resins, and hydrogenated terpene resins are desirable. Furthermore, the softening temperature of the aforementioned resin is not especially limited; it may be a solid or a liquid at room temperature, but liquid ones have better effects as plasticizers. Furthermore, if the quantity of the aforementioned tackifying resin ingredient in the composition is too great, the hand will become hard and the room-temperature adhesiveness will become extremely poor; if it is too small, the wettability with respect to the materials being adhered will be reduced, and the initial adhesive force will become poor. Therefore, the quantity of at least one kind of tackifying resin ingredient in the compound must be 40-70 wt %, preferably 50-70 wt %, and still more preferably 55-65 wt %.

The plasticizing oil ingredient used in this invention may be any one which is ordinarily used in hot melt adhesive compositions, but it is preferable to use those which have lower molecular weights than the aforementioned tackifying resin ingredient and which play the role of viscosity regulators, as well as increasing the low-temperature tackiness and softening the composition. As such plasticizing oil ingredients, oils which combine paraffin, naphthene, and aromatic ingredients are ordinarily used, but if the proportion of the paraffin ingredient is large, the thermal stability is increased, and if the proportion of the naphthene ingredient is large, the low-temperature adhesiveness is improved. Furthermore, if the proportion of the aforementioned plasticizing oil is too small, the hand becomes hard and the low-temperature adhesiveness is reduced; if it is too great, the high-temperature adhesiveness is reduced. Therefore, it must be 10-50 wt %, preferably 20-50 wt %, and still more preferably 20-40 wt %.

Besides the aforementioned necessary ingredients, the hot melt adhesive composition of this invention may also contain various kinds of additives which are used in conventional hot melt adhesive compositions. These additives include hindered phenol stabilizers, for improving the heat resistance, oxidation resistance, and photo-stability of the adhesive, ultraviolet ray absorbers, etc. When the aforementioned hindered phenol stabilizers are used, the quantity compounded should be 0.1-1 wt %. When the aforementioned ultraviolet ray absorbers are used, the quantity compounded should be 0.1-0.5 wt %.

Other synthetic rubber ingredients and resin ingredients which are ordinarily used in hot melt adhesive compositions may also be compounded in this adhesive for the purpose of regulating the softness, wettability, adhesiveness, melt viscosity, and softening point, as well as reducing the cost. Such optional ingredients include, for example, synthetic rubbers such as SIS and SBS; ethylene copolymers such as ethylene-vinyl acetate copolymers and ethylene-ethyl acrylate copolymers; and olefin resins such as atactic polypropylene, isotactic polypropylene, propylene-1-butene copolymers, propylene-1-butene-ethylene triple copolymers, etc. Moreover, liquid polybutene, liquid polyisobutene, liquid polyisoprene, or other softeners may also be compounded in this adhesive. The proportions of these optional ingredients in the composition should ordinarily be 20 wt % or less in colorless or white adhesives, and 15 wt % or less in colored products. Furthermore, since these optional ingredients tend to hinder the excellent properties obtained from the aforementioned necessary ingredients, their quantities should be kept as small as possible.

The hot melt adhesive composition of this invention can be obtained by, for example, putting the aforementioned raw materials into a melting, stirring, and mixing pot and heating while stirring.

The following examples further illustrate certain preferred embodiments of the invention and are not intended to be illustrative of all embodiments.

Examples 1-12

The raw materials shown in Table 1 below were compounded according to the compositions shown below, and melted and kneaded at approximately 150°C, to obtain the hot melt adhesive compositions desired.

## Table 1

| Component | | Grade / Supplier | 1 | 2 | 3 | 4 | 5 | 6 | 7 | 8 | 9 | 10 | 11 | 12 |
|---|---|---|---|---|---|---|---|---|---|---|---|---|---|---|
| Synthetic Rubber | SEPS | KL-2063 (Kuraray Co.) MI-30g/10 min St-13wt% | — | — | — | 20 | 10 | 5 | — | 10 | 20 | — | — | 15 |
| | | KL-2043 (Kuraray Co.) MI-13g/10 min St-13 wt% | 5 | 15 | 30 | — | — | — | 15 | — | — | 20 | 10 | — |
| | SEBS | Kraton® G-1650 (Shell) St-30wt% | 10 | 10 | 5 | 5 | 15 | 30 | 15 | — | — | 10 | — | — |
| | | Kraton® G-1652 (Shell) St-30wt% | — | — | — | — | — | — | — | 10 | 10 | — | 20 | 20 |
| Tackifier Resin | Hydrogenated Terpene Resin | Clearon M105 | 60 | 50 | 40 | 55 | — | — | 40 | 70 | — | — | — | 20 |
| | Hydrogenated Aromatic Resin | Arkon P100 | — | — | — | — | 45 | 35 | — | — | 50 | 50 | 30 | — |
| | Hydrogenated Aliphatic Resin | Eastotak H-100W | — | — | — | — | — | — | — | — | — | — | — | 20 |
| Plasticized Oil | Naphthene Type | Shellflex 371N (Shell) | 25 | 25 | 25 | 20 | 30 | 30 | — | — | 20 | 20 | 40 | 25 |
| | Paraffin Type | Kristol 352 (Exxon) | — | — | — | — | — | — | 30 | 10 | — | — | — | — |
| Antioxidant | Irganox® 1010 (Ciba Geigy) | | 1 | 1 | 1 | 1 | 1 | 1 | 1 | 1 | 1 | 1 | 1 | 1 |
| Ultraviolet Absorber | Tinuvin® P (Ciba Geigy) | | 0.5 | 0.5 | 0.5 | 0.5 | 0.5 | 0.5 | 0.5 | 0.5 | 0.5 | 0.5 | 0.5 | 0.5 |
| total | | | 101.5 | 101.5 | 101.5 | 101.5 | 101.5 | 101.5 | 101.5 | 101.5 | 101.5 | 101.5 | 101.5 | 101.5 |

EXAMPLES

Clearon M105=Yasuhara Chem.
Arkon P-100=Arakawa Chem.
Eastotak H-100W=Eastman Kodak

The properties of the aforementioned actual example articles were measured by the methods described below, and the results are shown in Table 2 below.

<Initial adhesive force>

The hot melt adhesive compositions, applied to PET films to a thickness of 50 $\mu$m in a 25°C atmosphere, were stuck to underwear underlayers in an area 2.54 cm × 20 cm, a load of 1 kg was applied, and this was left standing for 1 hour. The peeling strength was then measured with a tensile tester (according to JIS Z 0237).

<Adhesive force over time>

Test pieces made in the same manner as in the aforementioned adhesive force measurement were left standing for 8 hours with a 1 kg weight in a 40°C atmosphere. The peeling strength was then measured with a tensile tester (according to JIS Z 0237).

<Ability of adhesive to be peeled from underlayer>

After the aforementioned test of the adhesive force over time was performed, the peeled surface of the underwear underlayer was examined. If no hot melt adhesive composition at all remained on it, the sample was graded ○; if some remained, it was graded Δ, and if all of the composition remained, it was graded X.

<Thermal stability>

(1) Hue

The hues of the hot melt adhesive compositions, after they were left standing for 72 hours in a 180°C atmosphere, were judged by eye. Those which were completely colorless or white were graded ◎; those which were almost colorless or close to white were graded ○; those which were somewhat colored were graded Δ; and those which were clearly colored were graded X.

(2) Odor

The odors of hot melt adhesive compositions which were given the same thermal histories as in the aforementioned hue observation were evaluated by a functional test (10 panel members). Those which were completely odorless were graded ◎; those which were almost odorless were graded ○; those which had some odor were graded Δ; and those which had clear odors were graded X.

## TABLE 2

| | | EXAMPLES | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| Initial Adhesive Force (at 25° C) | Silk underlayer | 400 | 400 | 450 | 400 | 500 | 450 | 450 | 350 | 450 | 400 | 350 | 400 |
| | Nylon underlayer | 300 | 400 | 450 | 400 | 400 | 450 | 400 | 350 | 450 | 400 | 300 | 300 |
| | Polyester underlayer | 300 | 400 | 350 | 350 | 300 | 400 | 300 | 350 | 450 | 400 | 300 | 400 |
| Adhesive force over time (at 40° C) | Silk underlayer | 400 | 400 | 500 | 400 | 500 | 400 | 350 | 350 | 400 | 300 | 350 | 400 |
| | Nylon underlayer | 350 | 350 | 400 | 400 | 400 | 400 | 400 | 350 | 450 | 300 | 350 | 400 |
| | Polyester underlayer | 350 | 350 | 400 | 300 | 300 | 350 | 350 | 300 | 400 | 300 | 350 | 400 |
| Peeling ability of adhesive underlayer | | ○ | ○ | ○ | ○ | ○ | ○ | ○ | ○ | ○ | ○ | ○ | ○ |
| Thermal ability | Hue (at 180° C) | ○ | ◎ | ◎ | ◎ | ○ | ○ | ◎ | ◎ | ◎ | ○ | ○ | ○ |
| | Odor (at 180° C) | ○ | ○ | ○ | ○ | ○ | ○ | ○ | ○ | ○ | ○ | ○ | ○ |

EP 0 428 017 B1

(Comparison Examples 1-13)

Thirteen comparison example articles with different composition proportions from the aforementioned actual examples were prepared. These compositions are shown in Table 3 below. The properties of these comparison example articles were evaluated in the same manner as in the aforementioned actual examples. The results are shown in Table 4 below.

Table. 3

| | | | Comparison Examples | | | | | | | | | | | | |
|---|---|---|---|---|---|---|---|---|---|---|---|---|---|---|---|
| | | | 1 | 2 | 3 | 4 | 5 | 6 | 7 | 8 | 9 | 10 | 11 | 12 | 13 |
| Synthetic Rubber | SEPS | KL-2063 (Kuraray) | — | — | — | — | — | 3 | 35 | — | 10 | — | — | 16 | — |
| | | KL-2043 (Kuraray) | — | — | 16 | — | — | — | — | 20 | — | 16 | 10 | — | 10 |
| | SEBS | Kraton G-1650 (Shell) | 20 | — | — | — | — | 20 | — | 3 | 35 | — | — | — | 5 |
| | | Kraton G-1652 (Shell) | — | 20 | — | — | — | — | 10 | — | — | 16 | 5 | 16 | — |
| | SIS | Kraton TR1107 (Shell) | — | — | — | 20 | — | — | — | — | — | — | — | — | — |
| | SBS | Kraton TR1102 (Shell) | — | — | — | — | 20 | — | — | — | — | — | — | — | 30 |
| Tackifier Resin | Hydrogenated Terpene Resin | Clearon M105 | 50 | 25 | 10 | 15 | 50 | — | — | — | — | 35 | 75 | — | — |
| | Hydrogenated Aromatic Resin | Arkon P100 | — | 20 | 45 | — | — | 47 | — | 47 | — | — | — | 58 | — |
| | Hydrogenated AliphaticResin | Eastn Tak H-100Γ | — | — | — | 40 | — | — | 30 | — | 30 | — | — | — | — |
| Plasticized Oil | Naphtene type | Shellflex 371N (Shell) | — | 35 | — | 25 | 30 | 30 | — | — | 30 | 35 | 10 | 7 | 55 |
| | Paraffin type | Kristol 352 (Exxon) | 30 | — | 30 | — | — | — | 30 | 10 | — | — | — | — | — |
| Antioxidant | | Irganox 1010 (Ciba Geigy) | 1 | 1 | 1 | 1 | 1 | 1 | 1 | 1 | 1 | 1 | 1 | 1 | 1 |
| Ultraviolet Absorber | | Tinuvin P (Ciba Geigy) | 0.5 | 0.5 | 0.5 | 0.5 | 0.5 | 0.5 | 0.5 | 0.5 | 0.5 | 0.5 | 0.5 | 0.5 | 0.5 |
| total | | | 101.5 | 101.5 | 101.5 | 101.5 | 101.5 | 101.5 | 101.5 | 101.5 | 101.5 | 101.5 | 101.5 | 101.5 | 101.5 |

Table. 4

| | | Comparison Examples | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Initial adhesion (g/25mm, at25°C) | to silk | 250 | 250 | 400 | 500 | 300 | 200 | 500 | 400 | 200 | 200 | 300 | 200 | 400 |
| | to nylon | 200 | 200 | 400 | 500 | 200 | 100 | 500 | 400 | 100 | 100 | 300 | 100 | 400 |
| | to polyester | 200 | 150 | 400 | 400 | 200 | 100 | 400 | 400 | 100 | 100 | 400 | 100 | 400 |
| Adhesion on aging (g/25mm, at40°C) | to silk | 400 | 350 | 500 | 500 | 350 | 400 | 400 | 400 | 350 | 200 | 400 | 300 | 150 |
| | to nylon | 350 | 350 | 400 | 500 | 250 | 350 | 400 | 350 | 300 | 150 | 300 | 350 | 100 |
| | to polyester | 300 | 200 | 500 | 500 | 250 | 350 | 450 | 350 | 300 | 100 | 300 | 300 | 100 |
| Peel off from clothes | | △ | △ | × | × | × | O | × | × | O | O | × | O | × |
| Heat Resistance (180°C × 12hrs) | colour | O | O | O | × | × | O | O | O | O | O | O | O | O |
| | odor | O | O | O | × | × | O | O | O | O | O | O | O | O |

EP 0 428 017 B1

From the results in Table 4 above, it can be seen that the comparison example articles show undesirable properties with respect to adhesiveness, peeling ability from the underlayer, or thermal stabilities (hue or odor). In contrast to them, the example articles, as shown in the results in Table 2 above, show excellent properties in all these items.

As shown above, the hot melt adhesive composition of this invention has excellent initial adhesiveness, so that when it is used to prevent slipping by being applied to part of the surface of a napkin, etc., slipping between the napkin, etc., and the underwear underlayer, etc., is completely prevented. Moreover, since this composition also has an excellent ability to peel away from the underlayer, no adhesive remains on the underlayer when the napkin, etc. is removed after use. Moreover, since hydrogenated tackifying resins are used, it has excellent thermal stability and there is no need to be concerned about color or odor even when the napkin, etc., is heated in the manufacturing process. Consequently, the hot melt adhesive composition of this invention is an ideal adhesive for preventing slipping of napkins, etc.

## Claims

### Claims for the following Contracting States : BE, DE, FR, GB, IT, SE

1. A hot melt adhesive composition, characterized in that it contains 5-30 wt.% styrene-ethylene-propylene-styrene (SEPS) block copolymer, the styrene content of the SEPS copolymer being from 10-40 wt.%, 5-30 wt.% styrene-ethylene butylene styrene (SEBS) block copolymer, the styrene content of the SEBS-copolymer being from 10-35 wt.%, 40-70 wt.% hydrogenated tackifying resin ingredient, and 10-50 wt.% plasticizing oil ingredient.

2. A hot melt adhesive composition, characterized in that it contain 5-15 wt.% styrene-ethylene-propylene-styrene block copolymer, 10-20 wt.% styrene-ethylene-butylene-styrene block copolymer, 55-65 wt.% hydrogenated tackifying resin ingredient, and 20-40 wt.% plasticizing oil ingredient.

3. A disposable absorbent article for absorbing fluids, with a body facing surface and a garment facing surface having coated on the garment facing surface an adhesive layer of a hot melt adhesive composition for preventing slipping, said adhesive composition comprising:

   5-30 wt.% styrene-ethylene-propylene-styrene (SEPS) block copolymer, the styrene content of the SEPS copolymer being from 10-40 wt.%,

   5-30 wt.% styrene-ethylene-butylene styrene (SEBS) block copolymer, the styrene content of the SEBS-copolymer being from 10-35 wt.%,

   40-70 wt.% hydrogenated tackifying resin, and

   10-50 wt.% plasticizing oil.

4. The disposable article of claim 3, wherein the adhesive contains from 55-65 wt.% of the hydrogenated tackifying resin.

5. The disposable absorbent article of claim 4, wherein the adhesive contains 5-15 wt.% styrene-ethylene-propylene-styrene block copolymer, 10-20 wt.% styrene-ethylene-butylene-styrene block copolmyer, and 20-40 wt.% plasticizing oil.

6. The disposable absorbent article of claim 3, wherein the hydrogenated tackifying resin is selected from the group consisting of hydrogenated aliphatic petroleum terpene resins.

7. The disposable absorbent article of claim 3, wherein the article is a diaper or sanitary napkin and the adhesive contains from 20-40 wt.% of plasticizing oil.

8. The disposable article of claim 3, wherein the adhesive contains from 0.1 to 1 wt.% of hindered phenol stabilizier.

### Claims for the following Contracting State : ES

1. A disposable absorbent article for absorbing fluids, with a body facing surface and a garment facing surface having coated on the garment facing surface an adhesive layer of a hot melt adhesive composition for preventing slipping, said adhesive composition comprising:

5-30 wt.% styrene-ethylene-propylene-styrene (SEPS) block copolymer, the styrene content of the SEPS copolymer being from 10-40 wt.%,

5-30 wt.% styrene-ethylene-butylene styrene (SEBS) block copolymer, the styrene content of the SEBS-copolymer being from 10-35 wt.%,

40-70 wt.% hydrogenated tackifying resin, and

10-50 wt.% plasticizing oil.

2. The disposable article of claim 1, wherein the adhesive contains from 55-65 wt.% of the hydrogenated tackifying resin.

3. The disposable absorbent article of claim 2, wherein the adhesive contains 5-15 wt.% styrene-ethylene-propylene-styrene block copolymer, 10-20 wt.% styrene-ethylene-butylene-styrene block copolmyer, and 20-40 wt.% plasticizing oil.

4. The disposable absorbent article of claim 1, wherein the hydrogenated tackifying resin is selected from the group consisting of hydrogenated aliphatic petroleum terpene resins.

5. The disposable absorbent article of claim 1, wherein the article is a diaper or sanitary napkin and the adhesive contains from 20-40 wt.% of plasticizing oil.

6. The disposable article of claim 1, wherein the adhesive contains from 0.1 to 1 wt.% of hindered phenol stabilizier.

## Patentansprüche

### Patentansprüche für folgende Vertragsstaaten : BE, DE, FR, GB, IT, SE

1. Heißschmelzklebemasse bzw. -zusammensetzung, dadurch gekennzeichnet, daß sie 5 bis 30 Gew.-% Styrol-Ethylen-Propylen-Styrol-Blockcopolymer (SEPS), dessen Styrolgehalt 10 bis 40 Gew.-% beträgt, 5 bis 30 Gew.-% Styrol-Ethylen-Butylen-Styrol-Blockcopolymer (SEBS), dessen Styrolgehalt 10 bis 35 Gew.-% beträgt, 40 bis 70 Gew.-% hydriertes klebrigmachendes Harz und 10 bis 50 Gew.-% plastifizierendes Öl enthält.

2. Heißschmelzklebemasse, dadurch gekennzeichnet, daß sie 5 bis 15 Gew.-% Styrol-Ethylen-Propylen-Styrol-Blockcopolymer, 10 bis 20 Gew.-% Styrol-Ethylen-Butylen-Styrol-Blockcopolymer, 55 bis 65 Gew.-% hydriertes klebrigmachendes Harz und 20 bis 40 Gew.-% plastifizierendes Öl enthält.

3. Absorbierender Wegwerfgegenstand zum Absorbieren von Flüssigkeiten mit einer dem Körper zugewandten Fläche und einer der Bekleidung zugewandten Fläche, der auf der der Bekleidung zugewandten Fläche mit einer Klebstoffschicht einer Heißschmelzklebemasse zum Verhüten eines Weggleitens beschichtet ist, wobei die Klebstoffzusammensetzung umfaßt:

5 bis 30 Gew.-% Styrol-Ethylen-Propylen-Styrol-Blockcopolymer (SEPS), dessen Styrolgehalt 10 bis 40 Gew.-% beträgt,

5 bis 30 Gew.-% Styrol-Ethylen-Butylen-Styrol-Blockcopolymer (SEBS), dessen Styrolgehalt 10 bis 35 Gew.-% beträgt,

40 bis 70 Gew.-% hydriertes klebrigmachendes Harz und

10 bis 50 Gew.-% plastifizierendes Öl.

4. Wegwerfgegenstand nach Anspruch 3, in dem der Klebstoff 55 bis 65 Gew.-% des hydrierten klebrigmachenden Harzes enthält.

5. Absorbierender Wegwerfgegenstand nach Anspruch 4, in dem der Klebstoff 5 bis 15 Gew.-% Styrol-Ethylen-Propylen-Styrol-Blockcopolymer, 10 bis 20 Gew.-% Styrol-Ethylen-Butylen-Styrol-Blockcopolymer und 20 bis 40 Gew.-% plastifizierendes Öl enthält.

6. Absorbierender Wegwerfgegenstand nach Anspruch 3, in dem das hydrierte klebrigmachende Harze aus der Gruppe ausgewählt ist, die aus hydrierten aliphatischen Erdölterpenharzen besteht.

**7.** Absorbierender Wegwerfgegenstand nach Anspruch 3, der eine Windel oder eine Hygienebinde ist und in dem der Klebstoff 20 bis 40 Gew.-% plastifizierendes Öl enthält.

**8.** Wegwerfgegenstand nach Anspruch 3, in dem der Klebstoff 0,1 bis 1 Gew.-% gehinderten Phenolstabilisator enthält.

**Patentansprüche für folgenden Vertragsstaat : ES**

**1.** Absorbierender Wegwerfgegenstand zum Absorbieren von Flüssigkeiten mit einer dem Körper zugewandten Fläche und einer der Bekleidung zugewandten Fläche, der auf der der Bekleidung zugewandten Fläche mit einer Klebstoffschicht einer Heißschmelzklebemasse zum Verhüten eines Weggleitens beschichtet ist, wobei die Klebstoffzusammensetzung umfaßt:
5 bis 30 Gew.-% Styrol-Ethylen-Propylen-Styrol-Blockcopolymer (SEPS), dessen Styrolgehalt 10 bis 40 Gew.-% beträgt,
5 bis 30 Gew.-% Styrol-Ethylen-Butylen-Styrol-Blockcopolymer (SEBS), dessen Styrolgehalt 10 bis 35 Gew.-% beträgt,
40 bis 70 Gew.-% hydriertes klebrigmachendes Harz und
10 bis 50 Gew.-% plastifizierendes Öl.

**2.** Wegwerfgegenstand nach Anspruch 1, in dem der Klebstoff 55 bis 65 Gew.-% des hydrierten klebrigmachenden Harzes enthält.

**3.** Absorbierender Wegwerfgegenstand nach Anspruch 2, in dem der Klebstoff 5 bis 15 Gew.-% Styrol-Ethylen-Propylen-Styrol-Blockcopolymer, 10 bis 20 Gew.-% Styrol-Ethylen-Butylen-Styrol-Blockcopolymer und 20 bis 40 Gew.-% plastifizierendes Öl enthält.

**4.** Absorbierender Wegwerfgegenstand nach Anspruch 1, in dem das hydrierte klebrigmachende Harze aus der Gruppe ausgewählt ist, die aus hydrierten aliphatischen Erdölterpenharzen besteht.

**5.** Absorbierender Wegwerfgegenstand nach Anspruch 1, der eine Windel oder eine Hygienebinde ist und in dem der Klebstoff 20 bis 40 Gew.-% plastifizierendes Öl enthält.

**6.** Wegwerfgegenstand nach Anspruch 1, in dem der Klebstoff 0,1 bis 1 Gew.-% gehinderten Phenolstabilisator enthält.

**Revendications**

**Revendications pour les Etats contractants suivants : BE, DE, FR, GB, IT, SE**

**1.** Composition adhésive thermofusible, caractérisée en ce qu'elle contient 5 à 30 % en poids d'un copolymère séquencé styrène-éthylène-propylène-styrène (SEPS), la teneur en styrène du copolymère SEPS allant de 10 à 40 % en poids, 5 à 30 % en poids d'un copolymère séquencé styrène-éthylène-butylène-styrène (SEBS), la teneur en styrène du copolymère SEBS allant de 10 à 35 % en poids, 40 à 70 % en poids d'un ingrédient consistant en une résine d'adhésivité hydrogénée, et 10 à 50 % en poids d'un ingrédient consistant en une huile plastifiante.

**2.** Composition adhésive thermofusible, caractérisée en ce qu'elle contient 5 à 15 % en poids d'un copolymère séquencé styrène-éthylène-propylène-styrène, 10 à 20 % en poids d'un copolymère séquencé styrène-éthylène-butylène-styrène, 55 à 65 % en poids d'un ingrédient consistant en une résine d'adhésivité hydrogénée, et 20 à 40 % en poids d'un ingrédient consistant en une huile plastifiante.

**3.** Article absorbant jetable destiné à l'absorption de fluides, avec une surface en contact avec le corps et une surface en contact avec les vêtements, revêtu sur la surface en contact avec les vêtements d'une couche d'un adhésif consistant en une composition adhésive thermofusible pour la prévention du glissement, ladite composition adhésive comprenant :
5 à 30 % en poids d'un copolymère séquencé styrène-éthylène-propylène-styrène (SEPS), la teneur en styrène du copolymère SEPS allant de 10 à 40 % en poids,
5 à 30 % en poids d'un copolymère séquencé styrène-éthylène-butylène-styrène (SEBS), la teneur en styrène du copolymère SEBS allant de 10 à 35 % en poids,
40 à 70 % en poids d'une résine d'adhésivité hydrogénée, et
10 à 50 % en poids d'une huile plastifiante.

4. Article jetable suivant la revendication 3, dans lequel l'adhésif contient 55 à 65 % en poids de la résine d'adhésivité hydrogénée.

5. Article absorbant jetable suivant la revendication 4, dans lequel l'adhésif contient 5 à 15 % en poids du copolymère séquencé styrène-éthylène-propylène-styrène, 10 à 20 % en poids du copolymère séquencé styrène-éthylène-butylène-styrène et 20 à 40 % en poids d'huile plastifiante.

6. Article absorbant jetable suivant la revendication 3, dans lequel la résine d'adhésivité hydrogénée est choisie dans le groupe consistant en résines terpéniques aliphatiques hydrogénées dérivées du pétrole.

7. Article absorbant jetable suivant la revendication 3, qui est une couche ou une serviette périodique, l'adhésif contenant 20 à 40 % en poids d'huile plastifiante.

8. Article jetable suivant la revendication 3, dans lequel l'adhésif contient 0,1 à 1 % en poids d'un stabilisant consistant en un phénol à encombrement stérique.

**Revendications pour l'Etat contractant suivant : ES**

1. Article absorbant jetable destiné à l'absorption de fluides, avec une surface en contact avec le corps et une surface en contact avec un vêtement, revêtu sur la surface en contact avec un vêtement d'une couche d'un adhésif consistant en une composition adhésive thermofusible pour la prévention du glissement, ladite composition adhésive comprenant :
5 à 30 % en poids d'un copolymère séquencé styrène-éthylène-propylène-styrène (SEPS), la teneur en styrène du copolymère SEPS allant de 10 à 40 % en poids,
5 à 30 % en poids d'un copolymère séquencé styrène-éthylène-butylène-styrène (SEBS), la teneur en styrène du copolymère SEBS allant de 10 à 35 % en poids,
40 à 70 % en poids d'une résine d'adhésivité hydrogénée, et
10 à 50 % en poids d'une huile plastifiante.

2. Article jetable suivant la revendication 1, dans lequel l'adhésif contient 55 à 65 % en poids de la résine d'adhésivité hydrogénée.

3. Article absorbant jetable suivant la revendication 2, dans lequel l'adhésif contient 5 à 15 % en poids d'un copolymère séquencé styrène-éthylène-propylène-styrène, 10 à 20 % en poids d'un copolymère séquencé styrène-éthylène-butylène-styrène et 20 à 40 % en poids d'une huile plastifiante.

4. Article absorbant jetable suivant la revendication 1, dans lequel la résine d'adhésivité hydrogénée est choisie dans le groupe consistant en résines terpéniques aliphatiques hydrogénées dérivées du pétrole.

5. Article absorbant jetable suivant la revendication 1, qui est une couche ou serviette périodique, article dans lequel l'adhésif contient 20 à 40 % en poids d'une huile plastifiante.

6. Article jetable suivant la revendication 1, dans lequel l'adhésif contient 0,1 à 1 % en poids d'un stabilisant consistant en un phénol à encombrement stérique.